# EUROPEAN PATENT SPECIFICATION

(11) **EP 2 073 771 B1**
(45) Date of publication and mention of the grant of the patent: **17.03.2010**
(21) Application number: 07817837.3
(22) Date of filing: 12.10.2007
(51) Int. Cl.: A61F 13/02

(54) **A WOUND DRESSING**
WUNDVERBAND
PANSEMENT

(30) Priority: 13.10.2006 DK 200601334
(43) Date of publication of application: 01.07.2009
(73) Proprietor: Coloplast A/S, 3050 Humlebæk (DK)
(72) Inventor: GUNDERSEN, Boerge, DK-3080 Tikoeb (DK); BOUGHERARA, Chaabane, DK-2000 Frederiksberg C (DK); FREIDING, Markus, DK-25286 Helsingborg (SE); CHRISTENSEN, Jaime Wael, DK-2500 Valby (DK)
(86) International application number: PCT/DK2007/000441
(87) International publication number: WO 2008/043364

(56) References cited:
- EP-A1- 0 352 086
- WO-A-01/60296
- WO-A-02/05737
- WO-A-91/01706
- WO-A-93/19710
- WO-A-98/31402
- WO-A-03/043553
- WO-A-2006/089551
- US-A- 5 244 457
- US-A- 5 556 375
- US-A1- 2003 088 202

## Description

### BACKGROUND OF THE INVENTION

### 1. Field of the Invention

The invention relates to a wound dressing, especially an absorbent wound dressing being suitable for handling exuding wounds.

Wound dressings with layers for absorbing body fluids are known in the art. Absorbent layers are provided for the uptake of body fluids, especially wound exudates, so as to enable the wound dressing to keep a constant moist environment over the wound site, and at the same time avoiding maceration of the skin surrounding the wound.

Typically, such dressings comprise an absorbent layer and a backing layer and optionally an adhesive layer for adhering the dressing to the skin. Hydrocolloid adhesives may be used, due to their skin-friendliness and ability of absorbing moisture. However, hydrocolloid adhesives tend to be rather stiff and have an excellent adhesive tack, which may render them difficult or even painful to remove from the skin.

Softer adhesives, e.g. gel adhesives such as polyurethane or silicone adhesives are skin-friendly and having good tack but are yet easy to remove. However, these adhesives may be so soft that the dressings may be flimsy and difficult to handle as well as the risk of rolling up along the edge portions or detaching from the skin when exposed to stress, may be high. Furthermore, the silicone adhesive is a cured adhesive, not a hot melt adhesive like e.g. hydrocolloid adhesives, which may give rise to difficulties of the production of dressings.

A frequent problem when treating exuding wounds is maceration. Usually the absorbent part of the dressing is optimized to substantially vertical absorption, so that the skin surrounding the wound is not exposed to the exudates in order to avoid maceration of this healthy, but fragile skin. However, these properties are limiting the absorption capacity of the dressing to the part of absorbent material being directly over the wound. Barrier cream/skin conditioning paste, such as zinc paste, may be used on the surrounding skin in order to avoid the maceration, but the paste will often inhibit both the adhesive tack of the dressing as well as the ability of absorbing exudates.

Highly exuding wounds are often treated with absorbent materials such as foam or alginate, which is capable of absorbing high amounts of exudates but requires additional cover dressings, as well as the risk of maceration is high. Some absorbent materials, especially foam tends to expand in volume when absorbing moisture. The expansion of the absorbent material may induce severe stress in the dressing, that may lead to leakage or even detachment of the dressing as well as the risk of pressure sores may be high due to the pressure induced by the expanding foam.

When using a relatively weak adhesive like e.g. silicone gels, polyurethane gels or other gel adhesives it is important that the adhesive is not under too much stress as this may lead to unintended detachment. Therefore the construction needs to address this by the right combination of materials. Especially when using materials that expand during absorption it is important to handle any stress from the expanding materials. Hydrophilic foam used for wound dressings is susceptible to expansion during absorption.

Another aspect of using relatively week and skin friendly adhesive is the effective adhesive area and the use of adhesive is not always considered safe on the wound site. Therefore, when using an adhesive border the area need to be optimised so the dressing can stay in place under the condition under usage, therefore the skin adhesive is also extended over part of the foam.

Yet another aspect is the use of an absorber, which due to its thickness may impose pressure to the skin especially at the edge portions. Such pressure may give rise to pressure sores.

### 2. Description of the Related Art

In International patent application No. WO 02/05737 is disclosed a wound dressing comprising a backing layer, an adhesive layer and an absorbent layer there between. The adhesive layer is provided with a central aperture and the backing layer and the absorbent layer are not attached to each other. The adhesive layer may be provided with a reinforcing layer. The adhesive is preferably a hydrocolloid adhesive.

European patent application No. EP 633 758 discloses a wound dressing comprising a backing layer, an absorbent layer and an adhesive laminate. The laminate is in the form of a perforated film, coated with silicone adhesive on both surfaces. The adhesive has been provided with apertures coinciding with the perforations of the film. The adhesive laminate covers the entire wound-contacting surface.

European patent application No. EP 486 522 discloses a wound dressing comprising a foam layer, covered on one surface with a backing layer and on the other surface with an adhesive layer. The adhesive layer may be provided with a central aperture and an intermediate layer, in the form of a net, may be present between the foam and the adhesive layer.

US patent application No. 2003/0088202 A1 discloses an absorbent foam wound dressing having a wound facing base layer, a backing layer and a foam layer in between. The base layer is covered on the body side with suitable adhesive such as an acrylic adhesive, hydrogel or hydrocolloid adhesive. The opposite surface of the base layer is coated with a similar adhesive facilitating attachment to the foam and the backing layer. The base layer is provided with a central aperture enabling direct contact between the foam and the wound. The foam is preferably not attached to the backing layer rendering it possible for the foam to expand through the aperture and into the wound. The highly expanding foam exposes the dressing and especially the adhesive to severe stress and a strong adhesive is needed.

In International patent application No. WO 01/60296 is disclosed a wound dressing with a adhesive coated perforated base layer facing the wound, an absorbent layer and a backing layer. The absorbent layer is in the form of a hydrophilic gel.

### SUMMARY OF THE INVENTION

One object of the present invention is to provide a wound dressing with a high and fast initial absorption. The fast and high absorption of exudates including handling of viscous liquid reduces the occurrence of maceration.

Another object of the invention is to provide a wound dressing with a soft, skin-friendly adhesive, which has sufficient stiffness to be easy to apply and to avoid rolling up effects at the edge portions.

Still another object of the invention is to provide a dressing being capable of controlling an expanding absorbent element without detaching from the skin.

Yet another object is to avoid pressure marks from the edge of the foam layer.

A further object of the invention is to provide a dressing with good permeability.

A still further object is to provide a dressing that can be removed in one piece.

### Brief Description of the Drawings

The invention is disclosed more in detail with reference to the drawings in which
Figure 1 shows a cross-section (exploded view) of a dressing of the invention
Figure 2 shows another embodiment of the invention
Figure 3 shows the same embodiment of the invention when wetted,
Figure 4 shows another embodiment of the invention and
Figure 5 shows yet another embodiment of the invention.

### Detailed Description of the Present Invention

The invention relates to a wound dressing, said dressing comprising:
- a backing layer located on the non-skin-facing surface of the dressing,
- an adhesive laminate located on the skin-facing surface of the dressing, said laminate comprising
   a) a first discontinuous acrylate-based adhesive layer distal to the skin
   b) a support layer
   c) a second continuous silicone-based adhesive layer, proximal to the skin
- an absorbent foam layer, said foam layer is located between the backing layer and the adhesive laminate and the foam layer is attached to the backing layer and the adhesive laminate comprises an central aperture.

It is preferred that the area of the absorbent layer is smaller than the area of the backing layer, facilitating a flange portion of backing layer surrounding the absorbent layer as an "island" dressing.

The full coverage of a silicone adhesive facilitates better adhesion and has still good permeability. The permeability of the border part of the dressing is preferably higher than 250 gms/m²/24h and surprisingly does this relative low permeability function on skin. The relative low permeability is compensated by the high and fast initial absorption of the dressing combined with the high permeability over the central part over the foam pad.

By laminating by welding the foam layer to backing film a better permeability is achieved compared to adhesive lamination. The permeability of the central part of the dressing above the foam pad is preferably higher than 5000 gms/m²/24h, preferably higher than 7000 gms/m²/24h.

Fastening the backing layer to the foam layer facilitates better control of the foam when expanding so it will not distort or lift the soft silicone adhesive from the skin.

By using a silicone adhesive in a continuous layer as the skin-contacting adhesive, adhesion is improved compared to dressings with a pattern-coated adhesive. This improved adhesion renders it possible to provide the adhesive laminate with a central aperture over the wound and yet still remain enough adhesion to keep the dressing in place.

The dressing of the present invention may reduce the risk of pressure marks, induces by the edge portion of the foam layer. As the foam layer is located between the adhesive laminate and the backing layer, the edge portion of the foam is not in direct contact with the skin or wound site, but is protected by the adhesive laminate, the laminate is softening the edge portion of the foam layer.

The adhesive laminate has preferably an outer extent corresponding substantially to the backing layer, the edge portions being substantially coincident. The laminate is provided with a central aperture enabling direct contact between the wound and the absorbent layer. The aperture is preferably smaller than the absorbent layer, making the adhesive laminate cover at least the edge portion of the absorbent layer.

By the phrase "central aperture" is meant an adhesive-free area at the central portion of the adhesive laminate. Central should be interpreted as being in the middle portion of the dressing and not peripheral, but should not necessarily be symmetrically located on the dressing. The central aperture is preferably of a size corresponding to the wound, thus reducing the risk of maceration of the surrounding skin if exposed to a wet absorbent layer.

The non-skin-facing adhesive layer is an acrylate-based adhesive, preferably applied in a pattern. The acrylate acts as a construction adhesive, which adhere the backing layer to the support layer, and also secure the support layer to the foam at the edge portion of the foam layer. The acrylate adhesive is strong and adheres well to many surfaces and is thus suitable for holding the dressing together. The layer is discontinuous, e.g. applied in a pattern, in order to enhance the permeability of the layer.

The skin-facing adhesive layer is a silicone-based adhesive, applied in a continuous layer. The silicone adhesive is chosen for its skin-friendliness, it is soft and pleasant to wear and less painful to remove from the skin, compared to many other skin adhesives. However the softness of the adhesive also reduces the strength of the adhesive tack, which may be the reason for known dressings with silicone adhesive as skin contact layer usually are provided with a silicone adhesive layer without major interruptions such as a central aperture. However, the silicone adhesive of these known dressings is usually coated in a pattern in order to facilitate a higher permeability of the adhesive layer. In the present invention it has surprisingly been shown that having an adhesive skin-facing surface of in the form of a continuous layer of silicone adhesive, interrupted at the central portion by an aperture, a dressing with sufficient strength to withstand the stress from expanding foam is obtained.

In one embodiment of the invention the silicone-based adhesive is substituted by an adhesive gel comprising a polyalkyleneoxide polymer and organosiloxane based cross-linked adhesive system.

The overall adhesive surface needs to be large enough to secure the dressing to the skin and the aperture size defines an opening in the adhesive laminate.
The aperture area is preferably between 10-50% of the total dressing (wound facing surface) area, more preferred between 12-40% of the total dressing area.

The aperture area is preferably within a given range compared to the absorbent area, the aperture area may have a size to comply with the wound size, but not so large that one compromises the adhesion between the absorber and the support film. The overlap of the adhesive laminate at the rim of the absorbent layer is preferably between 3-20 mm.

The aperture area compared to the foam pad is between 40-80 %, preferably larger than 45%.

In one embodiment of the invention the adhesive laminate is provided with an adhesive-free zone surrounding the outer periphery of the absorbent layer. The non-adhesive zone is at least at the non-skin-facing surface of the adhesive laminate. Combined with moisture sensitive adhesive, the adhesive next to the absorbent layer may detach when wetted, and the detached part will be the adhesive area between the adhesive-free zone and the aperture, thus providing space for the absorbent layer to expand.

The non-adhesive zone may be achieved in different way, e.g. by lack of adhesive or by inhibiting the adhesion by applying a layer on the adhesive or applying a non-adhesive composition to the zone.

The non-adhesive zone has preferably a size of 5-20 mm, more preferred between 5-15 mm.

The backing layer may be any suitable layer, e.g. a polymer film, a non-woven a foam or a foamed film The backing layer has preferably a permeability greater than 1500 gms/m²/24h, more preferred greater than 5000 gms/m²/24h, most preferred greater than 10000 gms/m²/24h

In a preferred embodiment of the invention the backing layer is a polyurethane film. The film has preferably a thickness from 15-50 microns, more preferred between 20-40 microns.

The support layer may be any suitable layer, e.g. a polymer film, a non-woven a foam or a foam film.

In a preferred embodiment of the invention the support layer is a polyurethane film.

The support layer has preferably a thickness from 10-100 microns, more preferred between 20-40 microns. The permeability of the support layer is preferably greater than 800 gms/m²/24h, more preferred greater than 1000 gfns/m²/24h.

The construction adhesive is an acrylate adhesive and pattern coated in order to improve the permeability of the border part of the dressing. Alternatively a fully coated adhesive may be used if the permeability is high enough; the support layer with the adhesive should have a permeability greater than 1000 gms/m²/24h. The pattern of the adhesive coat is preferably interconnected to secure no leakage. -

The area of acrylate adhesive in the pattern should be high enough to laminate and keep the dressing together, but not so high that it will impact the permeability of the dressing. The adhesive coverage is preferably between 25-75 %, more preferably between 45-55%. The thickness of the acrylate adhesive is preferably between 5-30 microns, more preferred between 5-20 microns and most preferred between 7-15 microns.

Silicone based adhesives are very skin-friendly facilitating minimal pain and cell stripping during detachment. The silicone adhesive is very soft and does not have as powerful tack as e.g. acrylate adhesives. This means that it is more difficult to have a silicone adhesive to work in a dressing construction as it is more susceptible to stress fro e.g. an expanding foam. It has surprisingly been shown that by the dressing of the present invention the silicone adhesive as the second skin-facing adhesive is able to facilitate a secure attachment of the dressing to the skin.

The preferred adhesive for the second adhesive is a silicone gel, such as a platinum cured two-part system. The silicone gel adhesive layer has preferably a thickness between 100-400 microns, more preferred between 150 - 300 microns and most preferred between 200-300 microns.

The adhesive laminate is provided with a central aperture, providing a non-adhesive surface at the centre of the skin-facing surface of the dressing and enabling direct contact between the foam and the wound. A part of the absorbent layer is thus directly exposed to the wound. A high initial absorption is achieved, as the wound exudates do not have to pass an adhesive layer before entering the absorbent layer.

The location of the absorbent layer between the adhesive laminate and the backing layer reduces the risk of pressure marks from the edge portion of the absorbent layer to the skin. The presence of the adhesive laminate under the edge portion of the absorbent layer smooth out the edge and distributes the pressure.

Furthermore, by encapsulating the edge portion of the absorbent layer between the backing layer and the adhesive laminate the risk of leakage from the edge portion of the absorbent layer is eliminated.

In one embodiment of the invention the absorbent foam layer may be provided with a cover layer, covering the skin-facing surface of the foam layer. The cover layer may be any suitable layer with a high permeability. A preferred material for such layer is a perforated polyurethane film. The perforations facilitates high permeability for the exudates to enter the foam layer, and at the same time the cover layer controls the foam layer by keeping it in shape during expansion of the foam when wetted. In this way pressure marks arisen from the foam may be avoided as well as the dressing substantially retains it shape during use. The forces of the expanding foam are thus more easily controlled.

The cover layer may also act as a wound-contacting layer facilitating the wound does not stick to the foam layer.

In another embodiment of the invention the cover layer may only cover the edge portion of the foam layer, at least the portion in contact with the first acrylate adhesive. The cover layer may thus comprise an aperture corresponding to or being smaller that the aperture in the adhesive laminate. A better contact between the adhesive and the foam is thus achieved.

The cover layer is preferably laminated to the foam layer by welding.

Silicone-adhesives are very soft and a dressing comprising such adhesive may thus be difficult to handle during application. By introducing a support layer between two layers of adhesive, the adhesive laminate achieves more strength and stiffness and is thus easier to handle.

Furthermore, edge rolling is also reduced by using the adhesive laminate due to the improved stiffness specially the flexural modulus.

In order to ensure safe attachment to the skin the adhesive surface should be as large as possible, thus the adhesive laminate may extend over the edge portions of the foam.

The absorbent layer is preferably attached to the backing layer by welding. The welding can be on the whole backing layer or partly. The attachment renders it possible to better control the absorbent layer during production and during use. The absorbent layer may expand during wetting by wound exudates and the attachment to the backing layer prevents major buckles or folds of the absorbent layer. The part of the adhesive laminate covering the edge portion of the absorbent layer may also contribute to the control of the absorbent layer, as the edge portion of the absorbent layer thus is secured on both sides.

In one embodiment of the invention the first adhesive layer of the laminate may be sensitive to moisture, facilitating that the adhesive tack is reduced when wetted. When the absorbent layer is wetted, the absorbent layer releases from the adhesive laminate and the inner edge portion of the adhesive laminate, surrounding the aperture will not be exposed to stress and risk of rolling up, but the forces may instead be absorbed by the backing layer. Stress will be more homogenously distributed in the dressing and the risk of leakage or detachment of the dressing may be reduced.

The welding of the foam layer to backing layer and the acrylate adhesive bonding to the edge portion of the foam reduces the expansion of the foam and thereby the forces introduced in the adhesive.

When the acrylate adhesive layer releases from the foam when wetted the foam do not pull the silicone adhesive layer to detach from the skin.

The acrylate adhesive towards the absorbent layer may preferably be less moisture resistance than the silicone adhesive at the skin-facing surface, facilitating that the foam layer loosen from the adhesive thus reducing the forces at the skin adhesive.

The silicone adhesive may be casted directly onto the support layer in order to ensure proper attachment of the adhesive. The support film may comprise an adhesion promoter to secure anchorage between the silicone adhesive gel and the PU film.

The acrylate adhesive ensures strong attachment to support layer as well as it provides good permeability of the adhesive laminate. Furthermore, the strong acrylate adhesive facilitates good cohesiveness of the dressing, even when it is wetted, and removal in one piece is thus possible without leaving residues in the wound.

The laminated adhesive structure facilitates a good permeability; sufficient stiffness to secure easy handling, but yet soft and pleasant to wear as well as rolling up effect at the border portion is avoided.

The thickness of the adhesive laminate at the border is preferably between 0.2 - 0.8 mm, more preferred between 0.2 - 0.5 mm and most preferred between 0.25 - 0.4 mm.

The absorbent layer may have a thickness from 1-10 mm, more preferred from 1,5-8 mm and most preferred from 2-6 mm.

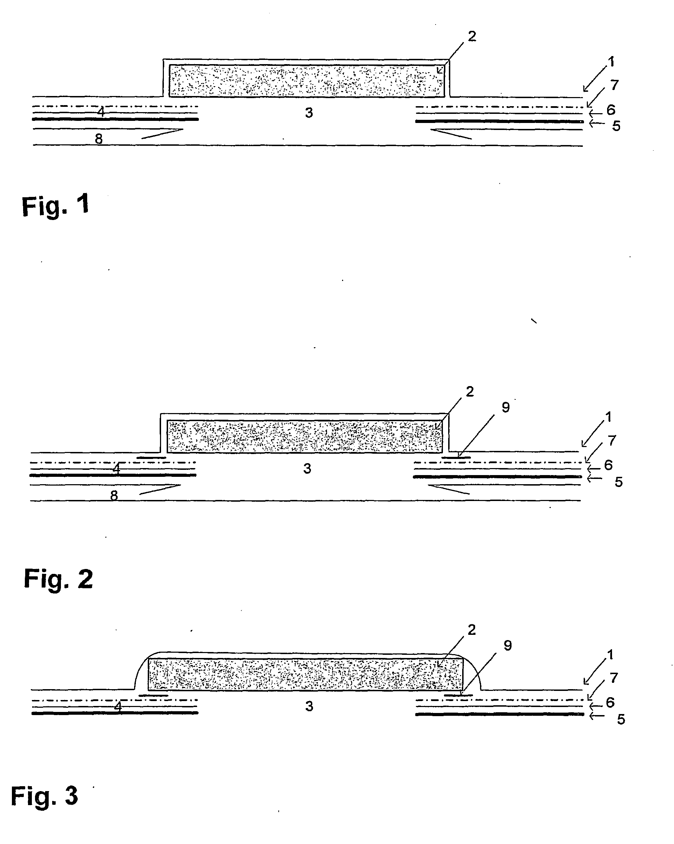

In another embodiment of the invention the first adhesive layer comprises two different adhesives. At the rim around the foam a more moisture sensitive adhesive may be used than at the border of the embodiment. The zone with the more moisture sensitive adhesive cover the area corresponding to the zone from the aperture edge to the non-adhesive zone shown in Figure 2.

The dressing of the present invention may comprise one or more active ingredients, for facilitation faster wound healing. The active ingredients may be biologically or pharmaceutically active agent.

The pharmaceutical medicaments will either be incorporated in the wound dressing or migrate to the wound surface and promote its function.

Examples of such pharmaceutical medicaments includes a cytochine such as a growth hormone or a polypeptide growth factor such as TGF, FGF, PDGF, EGF, IGF-1, IGF-2, colony stimulating factor, transforming growth factor, nerve stimulating growth factor and the like giving rise to the incorporation of such active substances in a form being apt to local application in a wound in which the medicament may exercise its effect on the wound, other medicaments such as bacteriostatic or bactericidal compounds, e.g. iodine, iodopovidone complexes, chloramine, chlorohexidine, silver salts such as sulphadiazine, silver nitrate, silver acetate, silver lactate, silver sulphate, silver sodium thiosulphate, silver-zirkonium complex or silver chloride, zinc or salts thereof, metronidazol, sulpha drugs, and penicillin's, tissue-healing enhancing agents, e.g. RGD tripeptides and the like, proteins, amino acids such as taurine, vitamins such ascorbic acid, enzymes for cleansing of wounds, e.g. pepsin, trypsin and the like, proteinase inhibitors or metalloproteinase inhibitors such as Illostat or ethylene diamine tetraacetic acid, cytotoxic agents and proliferation inhibitors for use in for example surgical insertion of the product in cancer tissue and/or other therapeutic agents which optionally may be used for topical application, pain relieving agents such as Ibuprofene or other NSAIDS, lidocaine or chinchocaine, emollients, retinoids or agents having a cooling effect which is also considered an aspect of the invention.

The active ingredient may also comprise odor controlling or odor reducing material.

### Description of the Preferred Embodiments

The invention is now explained more in detail with reference to the drawings showing preferred embodiments of the invention.

Figure 1 shows an embodiment of the invention with a backing layer (1) and a foam layer (2) attached to the backing layer (1) by welding. The foam layer (2) is smaller than the backing layer (1) so as to provide an island dressing where the foam layer (2) is surrounded of a border portion of the backing layer (1). The dressing is further provided with an adhesive laminate (4) covering the border portion of the backing layer (1) and extending also to cover the edge portion of the absorbent layer (2) thus leaving an adhesive-free aperture (3) at the central portion of the dressing. The adhesive laminate (4) comprises a skin-contacting layer of a silicone adhesive (5) in a continuous coat, a support layer (6), e.g. in the form of a polyurethane film, and a second adhesive layer (7) of an acrylate based adhesive, coated in a pattern of adhesive and non-adhesive zones. The adhesive surface may be protected by a release liner (8), e.g. siliconized paper or film, which is removed before application.

Figure 2 discloses another embodiment of the invention wherein the adhesive surface of the acrylate adhesive layer (7) is interrupted in a zone (9) encircling the foam layer (2). The non-adhesive zone (9) may be obtained either by providing an adhesive free zone or by inserting a non-adhesive layer, such as a film over the acrylate adhesive. In one embodiment of the invention the zone may be a cut-out through the entire adhesive laminate. When the foam layer (2) is wetted it begins to expand in volume, and the moisture reaches the acrylate adhesive (7) next to the foam layer (2), the acrylate adhesive loosens it tack and the piece of backing film next to the non-adhesive zone (9) is liberated to follow the movements of the foam layer (2), thus facilitating space for expansion, as shown in Figure 3. The forces induced from the expanding foam (2) are thus not directed into the adhesive laminate (4) but are accommodated in the backing layer (1).

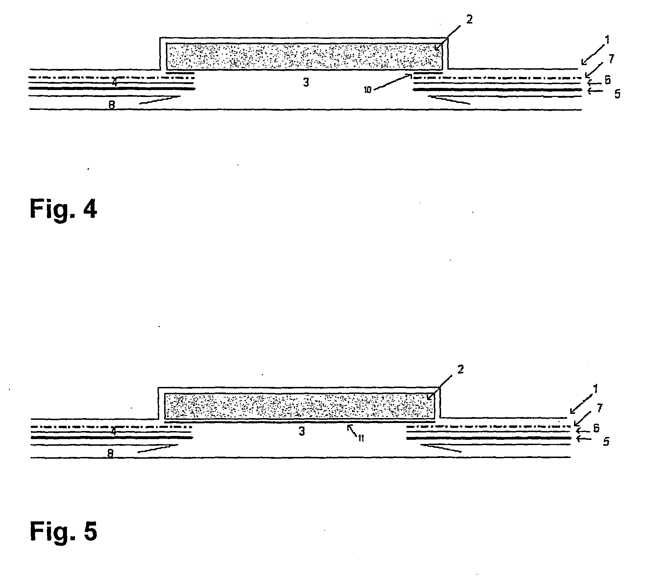

Figure 4 is shown an embodiment of the invention wherein the edge portion of the skin-facing surface of the foam layer (2) is provided with a cover layer (10). The cover layer (10) is laminated to the foam layer (2) on one surface and the acrylic adhesive (7) on the other surface. The cover layer ensures better contact between the adhesive (7) and the foam layer (2).

In Figure 2 is shown another embodiment of the invention wherein the cover layer (11) covers the entire skin-facing surface of the foam layer (7). The cover layer may be an apertured film. One surface of the cover layer (11) is laminated to the foam layer (7) by welding and the edge portion of the other surface of the cover layer (11) is adhered to the acrylic adhesive (7). In this embodiment the foam layer (2) is laminated to the backing layer (1) on one surface and the cover layer (11) on the other surface, and is in this way "boxed" in and large and undesired movements of the foam layer (7) during wetting and expansion may be prevented.

### MATERIALS AND METHODS

The embodiment can be produced in the following steps:
A support layer in the form of a PU film on a paper carrier is coated with the silicone gel in the preferred thickness e.g. by a knife over technology. The gel is cured in an oven at elevated temperature and a release liner is applied and the material is winded up on a roll.

In the next step the roll is un-winded and the paper carrier on the PU-film is removed and a pattern-coated acrylate is transfer coated from its release liner to the surface of the PU film, which is not coated with silicone gel. The adhesive laminate is winded up.

Alternatively the substrate for the silicone is a laminate consisting of a paper carrier, a pattern coated acrylate and a PU film.

In the next step the embodiment is assembled. One way is to have the non-touch as carrier, unwind the adhesive laminate and punch out the aperture. Remove the release liner from the silicone adhesive and laminate it to the non-touch, then remove the release liner from the acrylate. The absorbent foam pads are placed over the apertures and then the backing film is applied and the backing and foam is welded together. The carrier from the backing film is removed and the product is punched out.

Permeability test method:
The permeability of the materials is measured according to EN 13726-2

The backing material, the border adhesive, the central foam pad is tested with water contact acc. EN 13726-2, sec 3.3.

The support layer alone and the support coated with acrylate adhesive is tested from the support film side and with vapour contact according to N 13726-2, sec 3.2.

### Initial absorption methods:

The initial absorption speed can be measured by applying 0.1 ml of a liquid on the surface and measure the time to absorb. The liquid can be saline solution and more viscous liquid like artificial wound exudates.

The dressing ability to absorb larger amounts of liquid can also be measured by applying e.g. 1 ml liquid and again measure the time to fully absorbed.

## Claims

1. A wound dressing, said dressing comprising:
- a backing layer located on the non-skin-facing surface of the dressing,
- an adhesive laminate located on the skin-facing surface of the dressing, said laminate comprising
a) a first discontinuous acrylate-based adhesive layer distal to the skin
b) a support layer
c) a second continuous silicone-based adhesive layer, proximal to the skin
- an absorbent foam layer, said foam layer is located between the backing layer and the adhesive laminate and the foam layer is attached to the backing layer and the adhesive laminate comprises an central aperture.

2. A dressing according to claim 1 wherein the aperture constitutes at least 50 % of the skin-facing surface of the foam layer.

3. A dressing according to any of the preceding claims wherein the adhesive laminate at least covers the edge portion of the foam layer.

4. A dressing according to any of the preceding claims wherein the foam layer is attached to the adhesive laminate.

5. A dressing according to any of the preceding claims wherein the border portion of the dressing has a thickness of at least 0.2 mm.

6. A dressing according to any of the preceding claims wherein the surface area of the foam layer is smaller then the surface area of the backing layer.

7. A dressing according to any of the preceding claims wherein the edge portion of the adhesive corresponds to the edge portion of the backing layer.

8. A dressing according to any of the preceding claims wherein the first adhesive layer is provided with an adhesive-free zone circumferending the foam layer.

9. A dressing according to any of the preceding claims wherein the backing layer is a polyurethane film.

10. A dressing according to any of the preceding claims wherein the support layer is a polyurethane film.

11. A dressing according to any of the preceding claims wherein the first adhesive is moisture sensitive.

12. A dressing according to any of the preceding claims wherein the backing layer is laminated by welding to the foam layer.

13. A dressing according to any of the preceding claims wherein the first acrylate-based adhesive comprises tow different adhesives.

14. A dressing according to nay of the preceding claims wherein the foam layer is provided with a cover layer over the wound-facing surface.

15. A dressing according to claim 14 wherein the cover layer is an apertured film.

## Patentansprüche

1. Wundverband, der aufweist:
- eine Rückschicht, die auf der nicht zur Haut hin liegenden Oberfläche des Verbands angeordnet ist,
- ein Kleberlaminat, das auf der zur Haut hin liegenden Oberfläche des Verbands angeordnet ist und aufweist:
a) eine erste, distal zur Haut liegende diskontinuierliche Kleberschicht auf Acrylatbasis,
b) eine Trägerschicht,
c) eine zweite, proximal zur Haut liegende kontinuierliche Kleberschicht auf Siliconbasis,
- eine absorbierende Schaumschicht, die zwischen der Rückschicht und dem Kleberlaminat angeordnet und an der Rückschicht angebracht ist, wobei das Kleberlaminat eine zentrale Öffnung aufweist.

2. Verband nach Anspruch 1, bei dem die Öffnung mindestens 50 % der zur Haut hin liegenden Oberfläche der Schaumschicht darstellt.

3. Verband nach einem oder mehreren der vorhergehenden Ansprüche, bei dem das Kleberlaminat zumindest den Randbereich der Schaumschicht abdeckt.

4. Verband nach einem oder mehreren der vorhergehenden Ansprüche, bei dem die Schaumschicht am Kleberlaminat angebracht ist.

5. Verband nach einem oder mehreren der vorhergehenden Ansprüche, bei dem der Randbereich des Verbands eine Dicke von mindestens 0,2 mm aufweist.

6. Verband nach einem oder mehreren der vorhergehenden Ansprüche, bei dem die Oberfläche der Schaumschicht kleiner ist als die Oberfläche der Rückschicht.

7. Verband nach einem oder mehreren der vorhergehenden Ansprüche, bei dem der Randbereich des Klebers dem Randbereich der Rückschicht entspricht.

8. Verband nach einem oder mehreren der vorhergehenden Ansprüche, bei dem die erste Kleberschicht mit einer kleberfreien Zone versehen ist, welche die Schaumschicht umgibt.

9. Verband nach einem oder mehreren der vorhergehenden Ansprüche, bei dem die Rückschicht ein Polyurethanfilm ist.

10. Verband nach einem oder mehreren der vorhergehenden Ansprüche, bei dem die Trägerschicht ein Polyurethanfilm ist.

11. Verband nach einem oder mehreren der vorhergehenden Ansprüche, bei dem der erste Kleber feuchtigkeitsempfindlich ist.

12. Verband nach einem oder mehreren der vorhergehenden Ansprüche, bei dem die Rückschicht durch Verschweißen mit der Schaumschicht laminiert ist.

13. Verband nach einem oder mehreren der vorhergehenden Ansprüche, bei dem der erste Kleber auf Acrylatbasis zwei verschiedene Kleber umfasst.

14. Verband nach einem oder mehreren der vorhergehenden Ansprüche, bei dem die Schaumschicht auf der ganzen zur Wunde hin liegenden Oberfläche mit einer Deckschicht versehen ist.

15. Verband nach Anspruch 14, bei dem die Deckschicht ein mit Öffnungen versehener Film ist.

## Revendications

1. Pansement pour plaies, ledit pansement comprenant :
- une couche de renfort placée sur la surface du pansement qui n'est pas en contact avec la peau,
- un film adhésif placé sur la surface du pansement qui est en contact avec la peau, ledit film comprenant :
a) une première couche adhésive discontinue à base d'acrylate qui est distale par rapport à la peau
b) une couche de support
c) une seconde couche adhésive continue à base de silicone qui est proximale par rapport à la peau,
- une couche de mousse absorbante, ladite couche de mousse est placée entre la couche de renfort et le film adhésif, la couche de mousse est fixée sur la couche de renfort et le film adhésif comporte une ouverture centrale.

2. Pansement selon la revendication 1 dans lequel l'ouverture représente au moins 50 % de la surface de la couche de mousse en contact avec la peau.

3. Pansement selon l'une quelconque des revendications précédentes dans lequel le film adhésif recouvre au moins le bord de la couche de mousse.

4. Pansement selon l'une quelconque des revendications précédentes dans lequel la couche de mousse est fixée sur le film adhésif.

5. Pansement selon l'une quelconque des revendications précédentes dans lequel la partie constituant la bordure du pansement possède une épaisseur d'au moins 0.2mm.

6. Pansement selon l'une quelconque des revendications précédentes dans lequel la zone constituant la surface de la couche de mousse est plus petite que la zone constituant la surface de la couche de renfort.

7. Pansement selon l'une quelconque des revendications précédentes dans lequel le bord de l'adhésif correspond au bord de la couche de renfort.

8. Pansement selon l'une quelconque des revendications précédentes dans lequel la première couche adhésive est dotée d'une zone dépourvue d'adhésif sur la circonférence de la couche de mousse.

9. Pansement selon l'une quelconque des revendications précédentes dans lequel la couche de renfort est un film de polyuréthane.

10. Pansement selon l'une quelconque des revendications précédentes dans lequel la couche de support est un film de polyuréthane.

11. Pansement selon l'une quelconque des revendications précédentes dans lequel le premier adhésif est sensible à l'humidité.

12. Pansement selon l'une quelconque des revendications précédentes dans lequel la couche de renfort est feuilletée en soudant la couche de mousse.

13. Pansement selon l'une quelconque des revendications précédentes dans lequel le premier adhésif à base d'acrylate est constitué de deux adhésifs différents.

14. Pansement selon l'une quelconque des revendications précédentes dans lequel la couche de mousse est dotée d'une couche de protection sur la surface en contact avec la plaie.

15. Pansement selon la revendication 14 dans lequel la couche de protection est un film perforé.
